# EUROPEAN PATENT APPLICATION

(11) **EP 2 277 862 A2**
(43) Date of publication of application: **26.01.2011**
(21) Application number: 09735362.7
(22) Date of filing: 20.04.2009
(51) Int. Cl.: C07D 209/42, C07D 401/10, C07D 403/10, C07D 413/10, A61K 31/404

(54) **5-HYDROXY-4-AMINOMETHYL-1-CYCLOHEXANE OR (CYCLOHEPTYL)--3-ALKOXYCARBONYL INDOLE DERIVATIVES, PHARMACEUTICALLY ACCEPTABLE ANTIVIRAL SALTS THEREOF AND A METHOD FOR THE PRODUCTION THEREOF**

(30) Priority: 23.04.2008 RU 2008115405
(71) Applicant: Obschestvo S Ogranichennoy Otvetstvennostju "Binatekh", Kaluzhskaya obl. 249031 (RU)
(72) Inventor: VERKHOVSKY, Jury Grigorievich, Kaluzhskaya obl. 249032 (RU); TSYSHKOVA, Nina Gavrilovna, Kaluzhskaya obl. 249032 (RU); ROZIEV, Rakhimdzhan Akhmetdzhanovich, Kaluzhskaya obl. 249031 (RU); TSYB, Anatoliy Fedorovich, Kaluzhskaya obl. 249039 (RU); GONCHAROVA, Anna Yakovlevna, Kaluzhskaya obl. 249031 (RU); TROFIMOV, Fedor Alexandrovich, Kaluzhskaya obl. 249034 (RU); PODGORODNICHENKO, Vladimir Konstantinovich, Kaluzhskaya obl. 249032 (RU)
(74) Representative: Reichert, Werner Franz
(86) International application number: PCT/RU2009/000184
(87) International publication number: WO 2009/131493

(57) **Abstract**

The invention relates to novel 5-hydroxy-4-aminomethyl-1-cyclohexane or (cycloheptyl)--3-alkoxycarbonyl indole derivatives of general formula (I) and to pharmaceutically acceptable salts thereof exhibiting antiviral activity and to a method for the production thereof. The compounds can be used for treating and/or preventing such viral diseases as pig and bird flu. The invention also relates to a method for producing the compounds of general formula (I), wherein X is hydrogen, chlorine or iodine, n=1 or 2, R₃ is C₁-C₅ alkyl, Alk is a C₁-C₆ alkyl group, R₁ and R₂ are independently selected from C₁-C₄ alkyl, mainly methyl, or the NR₁R₂ group means groups of formula (II).

## Description

The invention relates to a novel group of 5-hydroxy-4-aminomethyl-1-cyclohexyl(or cycloheptyl)-3-alkoxycarbonyl indole derivatives, as well as their pharmaceutically acceptable salts possessing antiviral activity. Said compounds can find application for the production of drugs for the prevention and treatment of viral diseases.

Known antiviral compounds and drugs based on them as a rule possess antiviral activity only in relation to specific viruses. Therefore, the search for and creation of novel compounds possessing antiviral activity and the creation of drugs based on them is vitally necessary.

The mechanism of action of contemporary antiviral agents consists in the blocking of one of the stages of reproduction of viruses in the cells of the virus carrier (man) that include the stage of attachment of the virus to the cell and penetration into it, the insertion of the virus's nucleic acid into the genome of the host cell, the synthesis of its own DNA and RNA, and the synthesis and assembly of the intrinsic proteins of the virus. The majority of available antiviral drugs are analogs of nucleosides. They are effective; however, after their use the rebound phenomenon, leading to exacerbation of the illness, is possible in some patients. In addition, the development of resistance of the viruses to them is characteristic of a number of compounds with antiviral activity.

At the present time, drugs that have compounds of the amantadine group (amantadine and remantadine) (RU 2214997) as the active component are widely used in medical practice for the treatment of influenza. However, such drugs have a side effect on the CNS. The search for biologically active compounds on the basis of which drugs can be created that would possess a novel mechanism of action are an important direction in search for novel antiviral drugs. These can be substances that are capable of modulating (stimulating or suppressing) the body's immune reactions. Such an agent is arbidol (RU 2033156 and RU 2033157), which is 1-methyl-2-phenylthiomethyl-3-carbethoxy-4-dimethylaminomethyl-5-oxy-6-bromindol. Arbidol is capable of stimulating various immunity system functions, and as has been demonstrated, exerts a prophylactic and therapeutic effect in viral infections. At the same time, arbidol is practically nontoxic and is well tolerated by healthy individuals and those suffering from influenza. However, arbidol is insufficiently efficacious against some strains of viruses, for example, against the viruses of influenza A and B.

There are a number of patent publications (EP 1731506 A1, JP-63-188665, and CN 1482118) relating to derivatives of 5-hydroxyindole-3-carboxylates that possess antiviral activity. The 5-hydroxyindole-3-carboxylates are closest to the compounds of the present invention, and their use for the production of drugs for the treatment and prevention of viral diseases such as hepatitis B and HIV infection are described in EP 1731506 A1.

However, the compounds of the present invention were not produced and they are not disclosed in any of these publications, nor is their use for the production of drugs for the treatment or prevention of viral infections such as influenza A and B, as well as avian influenza.

The objective of the proposed invention is a search for novel indole derivatives possessing immune-stimulating activity and simultaneously having high antiviral activity in relation to specific viruses, for example, the viruses of influenza A and B, as well as avian influenza.

According to the present invention, 1-cyclohexyl (or cycloheptyl)-3-alkoxycarbonyl-4-diaminomethyl-5-hydroxyindoles derivatives corresponding to the general formula (I) are proposed: 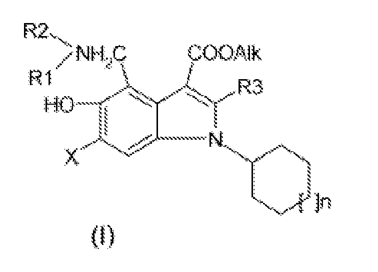
where X represents hydrogen, chlorine, iodine, and n = 1 or 2,
R₃ represents a C₁-C₃ alkyl,
Alk represents an C₁-C₆ alkyl group,
R₁, R₂ are independently selected from C₁-C₄-alkyl, primarily methyl, or R₁, R₂ together with a nitrogen atom (i.e., an NR₁R₂ group) represents groups corresponding to the formulas

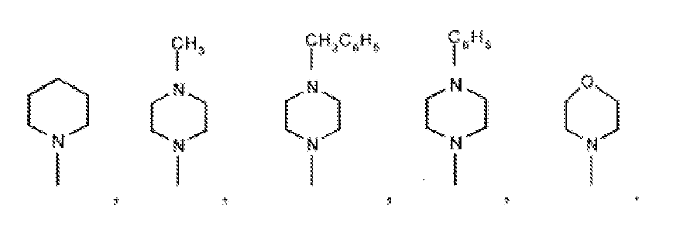

The invention also relates to pharmaceutically acceptable salts of said compounds of general formula I. The salts may be produced by the usual methods, for example, by treating compounds of formula (I) with the corresponding acids.

The pharmaceutically acceptable salts include hydrochlorides, mesylates, oxalates, and others.

A preferable compound is 5-hydroxy-4-dimethylamino-2-methyl-1-cyclohexyl-3-ethoxycarbonyl indole (compound A) and its hydrochlorides.

The compounds are synthesized in accordance with the scheme 1: 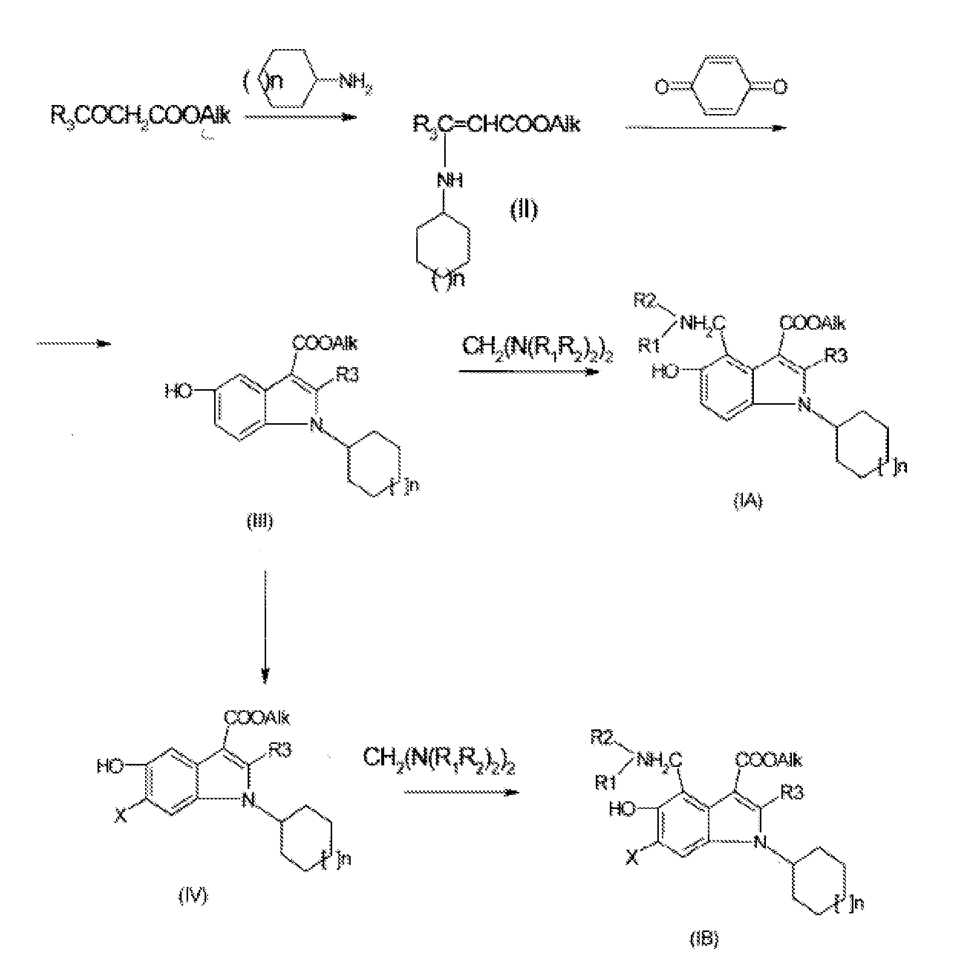 in said scheme R₁, R₂, R₃, X, Alk, and n have the above-mentioned meanings.

In accordance with said scheme 1, the method is implemented as follows:

In the interaction of an alkylcarbonylacetic ester of the formula

R₃COCH₂COOAlk

preferably acetoacetic ester, with cyclohexylamine or cycloheptylamine in the presence of a catalytic quantity of an acid, for example, hydrochloric acid, in a solvent medium, for example, benzol or toluol, the corresponding alkyl ester of β-cyclohexyl (or cycloheptyl) aminoalkenyl acid, primarily a C₁-C₆-alkyl ester of β-cyclohexyl (or cycloheptyl) aminocrotonic acid, of formula (II), is produced

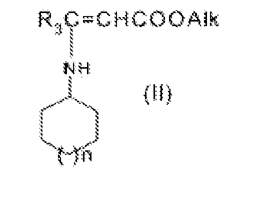

In the interaction of an ester of formula (II) with benzoquinone, 5-hydroxy-2-alkyl-1-cyclohexyl(or cycloheptyl)-3-alkoxycarbonyl indole of formula (III), is produced

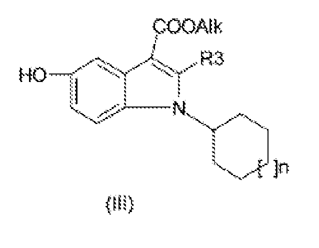

Then the compound of formula (III) is subjected to aminomethylation by the appropriate bisaminomethane of the general formula:

CH₂(N(R₁R₂)₂)₂

under conditions of the Mannich reaction, with the production of the corresponding 5-hydroxy-4-aminomethyl-2-alkyl-1-cyclohexyl (or cycloheptyl)-3-alkoxycarbonyl indole of formula IA:

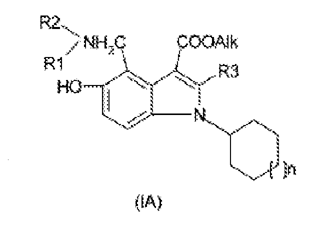

The meanings of the radicals R₁, R₂, R₃, X, Alk, and n in all structural formulas are those mentioned above.

With halogenation of a formula (III) compound, the corresponding 5-hydroxy-6-haloid-2-alkyl-1-cyclohexyl (or cycloheptyl)-3-alkoxycarbonyl indole of formula (IV), is produced

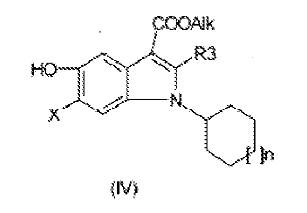

Compound (IV) is then aminomethylated by the appropriate compound of the general formula:

CH₂(N(R₁R₂)₂)₂

under conditions of the Mannich reaction, with the production of the corresponding 5-hydroxy-6-haloid-4-aminomethyl-2-alkyl-1-cyclohexyl (or cycloheptyl)-3-alkoxycarbonyl indole of formula IB:

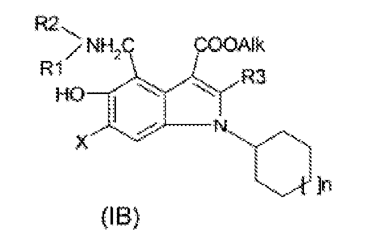

The meanings of the radicals R₁, R₂, R₃, X, Alk, and n are those mentioned above.

For the production of a formula IA or IB compound, where the NR₁R₂ group signifies groups corresponding to the formulas 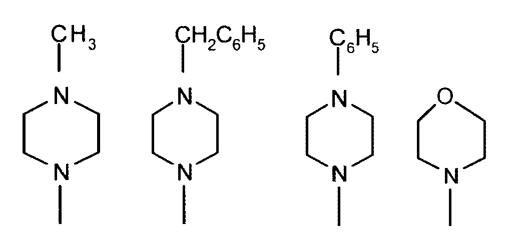 it is preferable to use the appropriate cyclic secondary amine and formaldehyde as the aminomethylating agent. For the production of a formula IA compound, where R₁ and R₂ each represent, for example, methyl, formaldehyde and the appropriate dialkylamine may also be used as the aminomethylating agent.

The products obtained are isolated in free form or in the form of pharmaceutically acceptable salts.

The compounds proposed according to the present invention are active in relation to different strains of influenza virus. At the same time, there are differences in efficacy of the proposed compounds and of the analog - Arbidol - in relation to different strains. The proposed compounds are also efficacious against other viruses and at the same time are not analogs of the nucleosides. This makes it possible to surmise that the compounds of this invention expand the list of compounds with a "mild" mechanism of action that does not affect the metabolic processes in the cells. In addition, the method of producing the compounds is simpler and proposes a smaller number of stages than in the production of known analogs. Therefore, the method of producing the proposed compounds is more economically advantageous.

At the same time, the method of producing the proposed compounds does not require the use of harsh and toxic starting compounds such as thiophenol.

The examples given below illustrate the proposed invention in greater detail.

The method of producing 5-hydroxy-4-dimethylaminomethyl-2-methyl-1-cyclohexyl-3-ethoxycarbonyl indole (A).

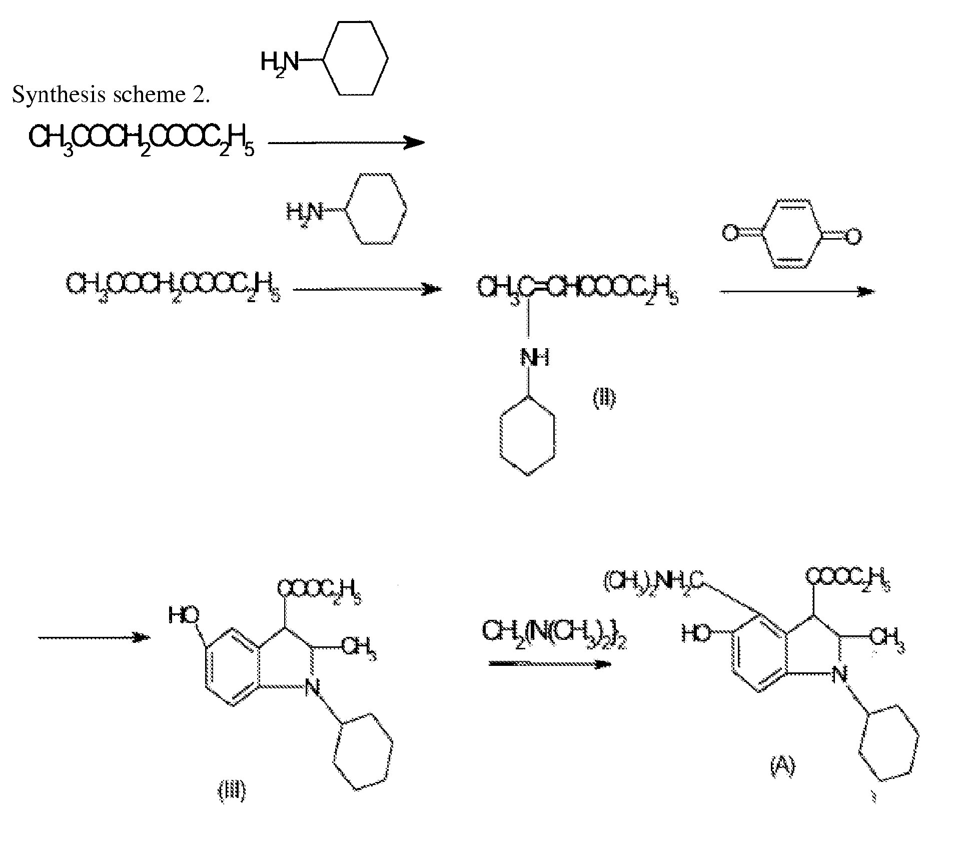

### Example 1. Ethyl ester of β-cylohexyl aminocrotonic acid (II).

To 13 g (0.1 mole) of acetoacetic ester in 20 mL of toluol are added 11.9 g (0.12 mole) of cyclohexylamine and 1 drop of concentrated HCl. Left at room temperature for 24 hours. The water liberated is then removed by means of a Dean-Stark trap. The toluol is evaporated in vacuum, the residue is sublimated. Boiling point 130-134°C /1 mm. nD²¹ 1.514. Yield of (II) 18.5 g (87%).

### Example 2. 5-hydroxy-2-methyl-1-cyclohexyl-3-ethoxycarbonyl indole (III).

To a solution of 2.1 g (0.01 mole) of (II) in 10 mL of glacial acetic acid are added 1.08 g (0.01 mole) 1,4-benzoquinone while mixing. The reaction mixture is left at room temperature for 24 hours. The precipitate formed is filtered, washed with cold acetic acid, and dried. Yield of (III) 0.6 g (20%), melting point 237°C (from acetic acid).

Found, %: C 71.14; H 7.87; N 4.78. C₁₈H₂₃NO₃. Calculated, C 71.74; H 7.69; N 4.65.

### Example 3. 5-hydroxy-4-dimethylaminomethyl-2-methyl-1-cylohexyl-3-ethoxycarbonyl indole (A).

A mixture of 1.2 g (0.004 mole) of (III) and 0.9 mL bis (dimethylamino)methane in 10 mL of dioxane is boiled for 4 hours. After distilling off the dioxane in vacuum, 5 mL of alcohol are added, cooled, and the precipitate filtered. Yield 1.0 g (69.7%), melting point 141-142°C (from alcohol).

Found, %: C 70.25; H 8.32; N 7.72. C₂₁H₃₀N₂O₃. Calculated: C 70.35; H 8.44; N 7.82.

The hydrochloride is produced by the addition of HCl in ether to a solution of base in acetone, melting point 194-195°C (from alcohol and ether).

Found, %: C 63.93; H 7.70; N 6.99. C₂₁H₃₁ClN₂O₃. Calculated, C 63.87; H 7.91; N 7.09.

The following are obtained under similar conditions with the use of the corresponding starting compounds:
5-hydroxy-4-dimethylaminomethyl-2-methyl-1-cycloheptyl-3-ethoxycarbonyl indole,
5-hydroxy-4-dimethylaminomethyl-2-methyl-1-cyclohexyl-3-ethoxycarbonyl indole,
6-chloro-5-hydroxy-4-dimethylaminomethyl-2-ethyl-1-cyclohexyl-3-ethoxycarbonyl indole,
6-iodo-5-hydroxy-4-dimethylaminomethyl-2-methyl-1-cyclohexyl-3-ethoxycarbonyl indole.
The salts of these compounds were obtained by treating the compounds with the appropriate acid.

The following compounds are obtained by using the corresponding starting compound (III), formaldehyde, and cyclic secondary amine under conditions of the Mannich reaction:
5-hydroxy-4-piperidinomethyl-2-methyl-1-cyclohexyl-3-ethoxycarbonyl indole and its hydrochloride,
5-hydroxy-4-morpholinomethyl-2-methyl-1-cyclohexyl-3-ethoxycarbonyl indole and its hydrochloride,
5-hydroxy-4-(N-methylpiperazino)methyl-2-ethyl-1-cyclohexyl-3-ethoxycarbonyl indole, and its hydrochloride and mesylate,
5-hydroxy-4-(N-benzylpiperazino)methyl-2-methyl-1-cyclohexyl-3-ethoxycarbonyl indole and its hydrochloride,
5-hydroxy-4-(N-phenylpiperazino)methyl-2-methyl-1-cyclohexyl-3-ethoxycarbonyl indole, and its oxalate and hydrochloride.

The salts of the compounds were obtained by treating the bases with the appropriate acid.

### The individuality of the products obtained was confirmed chromatographically in a benzene-alcohol, 10: 2, system on Silufol UV 254 plates.

The antiviral activity of these compounds was studied in vitro, and anti-influenza activity of these compounds was found in relation to influenza A viruses.

### Antiviral activity.

The effect of compound A on the influenza A virus was studied by the enzyme immunoassay (EIA) method according to the following scheme: the compound to be studied (preliminarily dissolved in ethyl alcohol and then in MEM nutrient medium) was applied to a monolayer of MDCK cells in a 96-well panel. To each virus control were added 100 µL of the same medium, and to each cell control - 200 µL. The panels were incubated at 37°C in a 5% CO₂ atmosphere for 2 hours.

To determine the effect of the antiviral preparations on the expression of viral antigens, ten-fold dilutions of the virus, taking into account the dilution by the preparation (1:5, 1:50, 1:500 etc.) in a volume of 100 µL of MEM medium, were added to the cells, after their incubation with compound A. The panels were incubated in an incubator in a 5% CO₂ atmosphere for 24 hours at 37°C.

The medium was removed, and the cells were fixed with 80% acetone in a PBS phosphate salt buffer for 15-20 minutes in a volume of 100 µL, and were then well dried and washed with PBS solution. Then to the cells were added 100 µL each of EIA buffer (phosphate salt buffer with 1% fetal serum and 0.05% TWEEN 20) and incubated at 37°C for 30 minutes. Following removal of the solution, 100 µL of monoclonal antibodies (MCAs) against the internal M and NP proteins of the influenza A virus were added to the cells in a 1:1000 dilution in EIA buffer. After incubation with the antibodies for 1 hour at a temperature of 37°C and with subsequent flushing twice, 100 µL of rabbit IgG against mouse IgG labeled with horseradish peroxidase in a 1:5000 dilution in EIA buffer were added to each well and incubated for 1 hour at 37°C. After flushing three times, the bound peroxidase was developed by the addition to the wells of 100 µL of a 0.05% solution of orthophenylene diamine (OPD) in 0.003% citrate buffer, pH 5.0, containing 0.003% H₂O₂ (subtractive buffer). The panels were kept for 15-30 minutes in the dark until the appearance of yellow coloration; the reaction was stopped by the addition of 50 µL of 1N H₂SO₄ to each well. Then the optical density (OD) was then measured on an automated spectrophotometer at a wavelength of 492 nm. Wells not loaded with the virus were used as the control.

The percent inhibition of virus reproduction by compound A was determined by the formula: percent inhibition = 100- (OD of the experiment - OD of the cell control) / (OD of the virus control without the compound - OD of the cell control) x 100. Three wells of the panel were used for one point of the experiment. The concentrations of the preparation or compound decreasing the OD value by 50% was taken to be the minimal inhibitory concentration (MIC₅₀).

**Table 1**

| Minimal Inhibitory Concentration (MIC₅₀) and Activity of Compound A in Relation to the Reference Influenza A/New Caledonia/20/99 (H1N1) Virus in in vitro Experiments | | | |
|---|---|---|---|
| Compounds | Solvent | MIC₅₀, µg/mL | Activity at 10 µg/mL, % |
| Arbidol | Alcohol | 6.0 | 89.5 |
| Compound A | Alcohol | 5.4 | 96.5 |

Determination of the efficacy of the compounds in the murine influenza pneumonia model.

The experiments were carried out on 60 white mice weighing 12-15 g. The animals were infected intranasally under light ether anesthesia with the influenza A/Aichi/2/69 (H3N2) virus (10 LD₅₀). The compounds to be tested were administered per os in a dose of 60 or 15 mg/kg/day. The treatment was carried out according to the following scheme: 24 hours and 1 hour before infection, and then 2 times a day (at 8 o'clock in the morning and 8 o'clock in the evening) for 5 days. The chemotherapeutic activity of Arbidol and compound A in the murine influenza pneumonia model was assessed according to two criteria: the index of protection against fatal viral infection and the increase in mean life span of the treated animals as compared with the control group. The mean life span of the mice was calculated according to the following formula: Σf(d-1)/n, where f is the number of mice dying on day d (the surviving mice were also included in f; d in this case = 16); n is the number of mice in the group.

**Table 2. Efficacy of Compound A as Compared with Arbidol in the Murine Influenza Pneumonia Model (A/Aichi/2/69 Virus).**

| Compounds | Survival rate | Mortality, % | Mortality reduction, % | Mean Life Span (Days) |
|---|---|---|---|---|
| Arbidol, 60 mg/kg/day | 6/10 | 40 | 50 | 10.8 |
| Arbidol, 15 mg/kg/day | 3/10 | 70 | 20 | 7.3 |
| Compound A, 15 mg/kg/day | 5/8 | 40 | 50 | 11.5 |
| Virus control (10 LD₅₀) | 1/10 | 90 | | 7.2 |

Thus, the results obtained attest to the virus-specific activity of the compounds applied for. Compound A in in vitro experiments proved to be somewhat more active than Arbidol (Table 1).

In animal experiments compound A was significantly more active than Arbidol. Thus the efficacy of compound A at a dose of 15 mg/kg/day was equal to the efficacy of Arbidol at a dose 4 times higher (60 mg/kg/day). At a dose of 15 mg/kg/day Arbidol was practically inactive (Table 2).

## Claims

1. Derivatives of 5-hydroxy-4-aminomethyl-1-cyclohexyl (or cycloheptyl)-3-alkoxycarbonyl indoles corresponding to the general formula (I) are proposed: 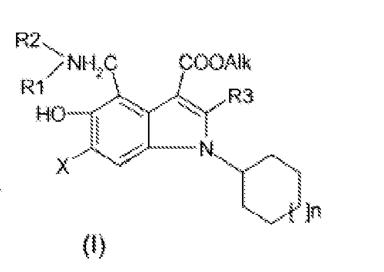
where X represents hydrogen, chlorine, iodine, and n = 1 or 2,
R₃ represents a C₁-C₃ alkyl,
Alk represents an C₁-C₆ alkyl group,
R₁, R₂ are independently selected from C₁-C₄-alkyl, primarily methyl, or an NR₁R₂ group represents groups corresponding to the formulas 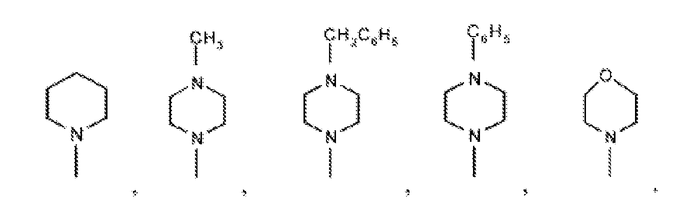 and the pharmaceutically acceptable salts thereof.

2. A compound according to claim 1, constituting 5-hydroxy-4-dimethylaminomethyl-2-methyl-1-cyclohexyl-3-ethoxycarbonyl indole.

3. A method for producing derivatives of 5-hydroxy-4-aminomethyl-1-cyclohexyl (or cycloheptyl)-3-alkoxycarbonyl indoles corresponding of the general formula (I) according to claim 1, consisting in the fact that a 5-hydroxy-2-alkyl-1-cyclohexyl (or cycloheptyl)-3-alkoxycarbonyl indole of formula (III) or (IV): 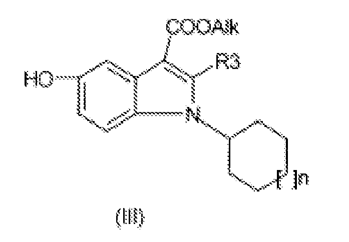 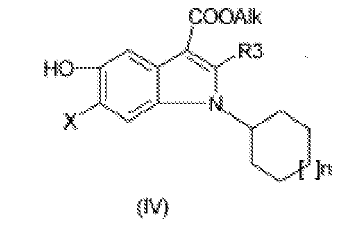
where the meanings of the radicals R₃, Alk, and n are indicated in claim 1,
is subjected to aminomethylation by a compound of general formula:
CH₂(N(R₁R₂)₂)₂
where R₁, R₂ are indicated in claim 1
under conditions of the Mannich reaction, with the production of the corresponding 5-hydroxy-4-aminomethyl-2-alkyl-1-cyclohexyl (or cycloheptyl)-3-alkoxycarbonyl indole of formula (1).

4. A method according to claim 3, distinguished by the fact that a compound of formula (III) is used, produced by the interaction of an appropriate alkylcarbonyl acetic ester of the formula
R₃COCH₂COOAlk
where the meaning of R₃ is indicated in claim 1
with cyclohexylamine or cycloheptylamine in the presence of a catalytic quantity of an acid in a solvent medium, to produce the corresponding alkyl ester of β-cyclohexyl (or cycloheptyl) aminovinylcarboxylic acid of formula (II) 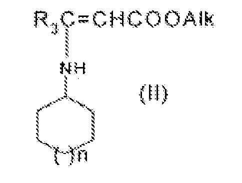
where the meanings of R₃, Alk, and n are indicated in claim 1,
which is subjected to the interaction with benzoquinone.

5. A method according to either claim 3 or 4, distinguished by the fact that acetoacetic ester is used as an alkylcarbonyl acetic ester, with the production of β-cyclohexyl (or cycloheptyl) aminocrotonic acid of formula (II), where R₃ means methyl.

6. A method according to either claim 3 or 4, distinguished by the fact that the compound of formula (III) is subjected to aminomethylation to produce compound (IA) 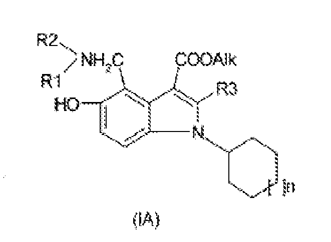 where the meanings of R₁, R₂, R₃, Alk, and n are indicated in claim 1.

7. A method according to either claim 3 or 4, according to which 5-hydroxy-4-dimethylaminomethyl-2-methyl-1-cyclohexyl-3-ethoxycarbonyl indole is produced.

8. A method according to either claim 3 or 4, distinguished by the fact that a compound of formula (IV), obtained by halogenation of a formula (III) compound, with subsequent aminomethylation, is used for the production of a compound of formula (IB): 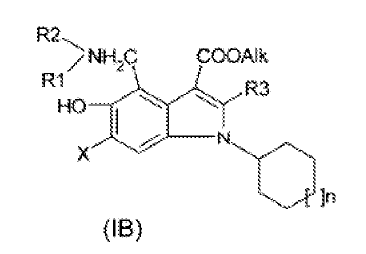 X means chlorine or iodine.
